# EUROPEAN PATENT SPECIFICATION

(11) **EP 3 967 300 B1**
(45) Date of publication and mention of the grant of the patent: **16.08.2023**
(21) Application number: 21195981.2
(22) Date of filing: 10.09.2021
(51) Int. Cl.: A61K 31/05, A61K 31/198, A61K 31/375, A61K 36/185, A61K 38/14, A61K 38/48, A61P 11/00, A61P 13/00, A61P 29/00, A61P 31/00, A61P 31/04

(54) **COMPOSITION FOR THE PREVENTION AND TREATMENT OF INFECTIONS AND/OR INFLAMMATIONS**
ZUSAMMENSETZUNG ZUR VORBEUGUNG UND BEHANDLUNG VON INFEKTIONEN UND/ODER ENTZÜNDUNGEN
COMPOSITION POUR LA PRÉVENTION ET LE TRAITEMENT D'INFECTIONS ET/OU D'INFLAMMATIONS

(30) Priority: 11.09.2020 IT 202000021562
(43) Date of publication of application: 16.03.2022
(73) Proprietor: Anvest Group S.r.l., 20152 Milano (IT)
(72) Inventor: ALFIERI, Sandro, 84083 Castel San Giorgio SA (IT); LUCCHEO, Luigi, 84083 Castel San Giorgio SA (IT); LUCCHEO, Gianni, 84086 Roccapiemonte SA (IT)
(74) Representative: Cattaneo, Elisabetta

(56) References cited:
- WO-A1-2016/120787
- MOYO MACK ET AL: "Medicinal properties and conservation of Pelargonium sidoides", JOURNAL OF ETHNOPHARMACOLOGY, ELSEVIER IRELAND LTD, IE, vol. 152, no. 2, 21 January 2014 (2014-01-21), pages 243-255, XP028661456, ISSN: 0378-8741, DOI: 10.1016/J.JEP.2014.01.009
- IKUTA KAZUFUMI ET AL: "Anti-viral and anti-bacterial activities of an extract of blackcurrants (Ribes nigrum L.)", MICROBIOLOGY AND IMMUNOLOGY, WILEY-BLACKWELL PUBLISHING ASIA, AU , vol. 56, no. 12 30 November 2012 (2012-11-30), pages 805-809, XP009516099, ISSN: 1348-0421, DOI: 10.1111/J.1348-0421.2012.00510.X Retrieved from the Internet: URL:https://api.wiley.com/onlinelibrary/td m/v1/articles/10.1111%2Fj.1348-0421.2012.0 0510.x [retrieved on 2012-11-29]
- FILARDO SIMONE ET AL: "Therapeutic potential of resveratrol against emerging respiratory viral infections", PHARMACOLOGY AND THERAPEUTICS., vol. 214, 17 June 2020 (2020-06-17), page 107613, XP055806360, GB ISSN: 0163-7258, DOI: 10.1016/j.pharmthera.2020.107613
- ZOYA MANZOOR ET AL: "Bromelain: Methods of Extraction, Purification and Therapeutic Applications", BRAZILIAN ARCHIVES OF BIOLOGY AND TECHNOLOGY, vol. 59, no. 0, 1 January 2016 (2016-01-01), XP055476530, DOI: 10.1590/1678-4324-2016150010

## Description

### FIELD OF THE INVENTION

The present invention relates to compositions or mixtures useful for the prevention and treatment of inflammatory symptoms and/or infections, in particular of the upper respiratory tract and/or urinary tract.

The present invention originates in the field of nutraceuticals, pharmaceuticals, and food supplements.

In particular, the present invention concerns compositions for oral administration suitable for the prevention and treatment of inflammatory symptoms and/or infections, in particular of the upper respiratory tract and urinary tract with biofilm production.

### STATE OF THE ART

The upper airways consist of nasal, paranasal, pharynx and larynx cavities.

The airways, from nose to bronchi, are moistened by a layer of mucus secreted by mucus cells present in the epithelium lining the airways which, in addition to humidifying the surfaces, traps the air particles and prevents most of it from reaching the alveoli. Mucus is a stringy and viscous substance, with adhesive properties, that protects the mucous membranes of the respiratory system, on which it forms a thin layer of viscoelastic gel. In physiological conditions, mucus is necessary to capture dust and microorganisms, while that produced under inflammatory stimuli is called phlegm, because it is more abundant than the norm, but it also has a different composition (being denser and more viscous).

Rhinosinusitis is an inflammation of the mucous membrane of the nose and paranasal sinuses, mainly caused by an allergy or a viral or bacterial infection.

Some of the more common symptoms of rhinosinusitis are pain, tenderness on palpation, nasal congestion, and headache. Sinusitis can affect any of the four groups of paranasal sinuses: maxillary, ethmoid, frontal, or sphenoid. It almost always develops in association with inflammation of the nasal cavities (rhinitis). It can be acute or chronic.

Laryngitis is inflammation of the larynx, usually due to viral (mainly) or bacterial proliferation.

Pharyngitis is inflammation of the mucous membrane of the pharynx, the back of the throat between the tonsils and the larynx. In most cases, pharyngitis is caused by viruses, more rarely by bacteria (streptococcus). The classic drug therapy indicated for pharyngitis is represented by antibiotic therapy, which however has the limit of being unable to reduce the viral load, which is the main cause of the disease. In addition, the bacterial colonies that can cause pharyngitis are coated and protected by a dense biofilm, which makes the antibiotic ineffective.

Sinusitis is called acute if it resolves completely in less than 30 days. In people with a functioning immune system, acute sinusitis is usually caused by a viral infection. In some cases, the etiological agent is a bacterium.

Sinusitis is defined as chronic when it lasts longer than 90 days. Causative factors include chronic allergies, nasal polyps, and exposure to environmental irritants.

The treatment of acute sinusitis is aimed at improving sinus drainage and resolving the infection. Washing with a saline solution (nasal irrigation) or the use of a nasal saline spray can help relieve symptoms. Decongestant nasal sprays, based on phenylephrine and oxymetazoline, can be used for a limited time. Corticosteroid nasal sprays can help relieve symptoms but take time to work.

External otitis, which affects the ear canal, is usually caused by bacterial or fungal infections or by eczema, and is favoured by external agents, such as cold, excessive humidity or dryness of the ear canal, accumulation of ear wax or incorrect maneuvers to remove the same, or contact with polluted water (swimming in a pool or in dirty water).

Otitis media affects the eardrum area and is often caused by respiratory tract infections that spread to the middle ear.

The pharmacological therapy aims to resolve the infectious process and prevent exacerbations and complications, and is based on the use of local or general antibiotics for about 10 days to ensure complete resolution of the infection although the pain in the ear disappears in a short time. Painkillers and anti-inflammatories can be used to relieve pain.

The urinary tract is a set of organs whose task is to filter the bloodstream from catabolites (waste substances) that accumulate therein, through urinary excretion. Urinary tract infections are divided into lower tract infections (affecting the urethra and bladder and called urethritis and cystitis, respectively); and upper tract infections (affecting ureters and kidneys). Urinary tract infections can be caused by bacteria (in the overwhelming majority of cases by *Escherichia coli*) and, less frequently, by fungi. All strains of *E*. *Coli* are able to anchor themselves to the tissue by means of Type 1 and Type P fimbriae. Type 1 fimbriae are defined as mannose-sensitive. The urovirulent *E*. *Coli* strains, which trigger pyelonephritis and urethritis, are equipped with additional fimbriae, called P, which anchor the bacteria to the tissue of the bladder.

The symptoms of urinary infections are: pollakiuria, dysuria, stranguria, fever.

In recurrent cystitis there is the formation of a bacterial biofilm by *Escherichia coli.* The biofilm produced by pathogenic microorganisms confers resistance to antibiotics and insensitivity to the immune system cells. The unpredictable reactivation of this reserve of bacteria justifies the recurrence and chronicization of these infections, which is why a decisive therapeutic strategy is required.

It is known that the term biofilm refers to an aggregate of microorganisms enclosed within a sort of mass endowed with a gelatinous consistency (also called slime) and made up of proteins, polysaccharides, and genetic materials. Thanks to its consistency, in fact, bacteria are able to attach themselves to the tissue surface forming numerous colonies; furthermore, the biofilm represents a shield, thanks to which bacteria are protected from the lethal action of antibiotics and our immune defenses. The biofilm role in relapsing and chronic infections is well known and relevant, considering that more than 60% of bacterial infections appear to be attributable to it.

In particular, the capacity to synthesise biofilm has been proven for the bacterial species most frequently involved in the pathogenesis of infectious processes of the upper airways, such as *Haemophilus influenzae, Streptococcus pneumoniae, Moraxella catarrhalis, Staphylococcus aureus* and *Pseudomonas aeruginosa.*

Different studies have shown the presence of bacterial biofilm inside the middle ear and at the level of the adenoid tissue in patients subject to acute otitis media, recurrent acute otitis media and chronic otitis media.

For acute sinusitis that is severe (3 or more days of symptoms such as fever equal to or higher than 39°C and severe pain) or persistent (for 10 or more days), antibiotics such as Amoxicillin/Clavulanate or Doxycycline are prescribed. Subjects with chronic sinusitis take the same antibiotics but for a longer period of time, typically 4 to 6 weeks. The limitation of antibiotics, however, appears evident in the case of bacterial infections with biofilm formation. In fact, it makes the colony unassailable by the drug.

It is also known that N-acetyl-l-cysteine (NAC) has antioxidant and mucolytic activity: the presence of a free thiol group (-SH) in the molecule is able to reduce and break disulphide bridges (-SS-) responsible for the aggregation of proteins and therefore for the high viscosity of the mucus. Therefore, the use of N-acetyl-I-cysteine and other cysteine derivatives as a mucolytic and fluidifying agent in the treatment of airway affections is known.

In addition, inside the cell NAC contributes to the synthesis of glutathione which plays an important role for cellular function in the defence against oxidizing agents and cytotoxic substances. In this non-mucolytic function thereof, NAC role is to restore the ratio between harmful oxidants and physiological anti-oxidants in favour of the anti-oxidants, through the ability to increase the levels of reduced glutathione, thus reducing oxidative damage to the respiratory system.

NAC is also used in the therapy for chronic infectious diseases of the respiratory tract due to biofilm-forming bacteria, as it inhibits bacterial adhesion and helps dissolve the biofilm matrix.

It is estimated that biofilm formation is involved in at least 60% of all chronic and/or recurrent infections and in 30% of exudates developed over the course of otitis media. Studies carried out with N-acetyl cysteine have shown to reduce bacterial adhesion and disintegrate the biofilm in different anatomical sites. The advantages offered by the administration of this mucolytic agent are linked not only to the reduction of clinical conditions characterized by the presence of abundant secretions, but also to the prevention of infectious-inflammatory exacerbation episodes in patients suffering from chronic bronchitis and COPD, due to its ability to increase the levels of reduced glutathione, with antioxidant action.

Equally well known are the properties of certain enzymes (proteases), such as bromelin, which appears to help fight the pain and swelling associated with inflammation.

In particular, proteases are able to thin the dense and viscous secretions thanks to their proteolytic action, without having side effects on the gastric system.

Bromelin is a set of enzymes, endowed with proteolytic activity, extracted from the pulp, but mostly from the stem, of pineapple. The anti-inflammatory, anti-edema, antithrombotic, anticoagulant and immunomodulating activities of bromelin are today the most used in the clinical environment. Bromelin inhibits thromboxane synthetase A2, an enzyme that converts PGH2 into pro-inflammatory molecules such as prostaglandins. The anti-inflammatory activity is also linked to an interaction with lipoxygenase, with reduction of the synthesis of mediators with phlogogenic and vasodilating action, such as Kininogen, Bradykinins, Thromboxane and Prostaglandins E2. It has antiplatelet and fibrinolytic activity. This action is essential for the anti-edema activity. It is particularly effective in the treatment of inflammatory conditions of the soft tissues associated with trauma, in localized inflammation in the presence of edema, and in postoperative tissue reactions. It is a protease capable of hydrolysing and inactivating the peptides that form many mediators of inflammation. Bromelin has mucolytic activity, useful in respiratory tract disorders; in particular, it has proved to be a good decongestant of the mucous membranes in case of acute catarrhal inflammation. These effects, consequent to the improvement of respiratory congestion, are due to the ability of Bromelin to thin and reduce bronchial secretions.

Pelargonium sidoides extracts have a strong immunomodulating and antiviral action, attributable to the phytocomplex thereof, particularly rich in active ingredients and useful for the treatment of colds. Pelargonium root extracts consist of carbohydrates and polysaccharides with immunomodulating and antiviral activities. They also include simple phenolic compounds (especially gallic acid), hydrolysable and condensed tannins with high molecular weight, flavonoids and minerals such as calcium and silicon, polyhydroxylated coumarins and polyphenols, including proanthocyanidins with anti-adhesive action against bacteria, no longer able to bind to the surface of the host cell to infect it. Furthermore, the increase in the ciliary beating of the nasal epithelial cells is at the basis of the motor secretory effect. Most airway infections are of viral origin, to which a bacterial infection can in some cases be added. In addition to non-specific immunomodulating and antiviral actions, the standardized alcoholic extract of Pelargonium sidoides has been tested for its ability to increase the production of nitrogen monoxide by macrophages, which in turn determines an increase in pro-inflammatory cytokines. (IL-6 and TNF-a) thus promoting chemotaxis, production of antibodies and increase of the Th1 response. The antibacterial activity was tested in vitro on *Klebsiella pneumoniae, Pseudomonas aeruginosa, Staphylococcus aureus, Proteus mirabilis.*

Resveratrol is able to modulate numerous physiological processes in the body. Polygonum cuspidatum is the major natural source of resveratrol, present in the most active biological form. In nature, resveratrol is synthesized by numerous plant species in response to physiological stimuli and external stress. The antiviral action of resveratrol has long been studied to understand the mechanism of action according to which the phytocomplex is able to inhibit viral replication. During viral infection, the virus nucleic acids are packaged in helical complexes by specific polymerases that "store" them in the host cell nucleus, and then export them to the cytosol at the time of maximum infection and generate other viruses. Resveratrol interferes with the synthesis of viral proteins thanks to the inhibition of the polymerases responsible for exporting viral RNA into the host cell. Resveratrol, like other polyphenols, has anti-inflammatory properties: by activating sirt1 it is able to inhibit the formation of a series of inflammatory factors in our body, including NF-kB, a transcription factor that increases the production of pro-inflammatory cytokines (such as TNF-alpha, IL-1 beta, IL-6) and cyclooxygenase (COX-1 and 2), i.e. the enzyme that transforms arachidonic acid into prostaglandins and inflammatory thromboxanes, by the cells of the immune system. The evidence that the function of resveratrol is partly mediated by Sirt1 is confirmed by the observation that the anti-inflammatory properties of resveratrol are abolished by genetic deletion of Sirt1 or addition of Sirt1 inhibitors. Another property of resveratrol is to reduce oxidative stress by increasing the production of antioxidant molecules such as glutathione S-transferase (GST), glutathione peroxidase (Gpx), NQO1, catalase and superoxide dismutase (SOD) (4). The mechanism by which resveratrol is able to increase the production of antioxidants in our body is the activation of the Nrf-2 gene expression. Resveratrol is considered to be more potent than vitamin E, one of the most potent antioxidants known to date.

Currant extract is rich in triterpenes and polyphenols, as well as flavonoids (in particular, Proanthocyanidins and Quercetin), capable of stimulating the production of cortisol by the adrenal glands (cortison-like action), exerting anti-inflammatory and antihistamine activities. In *in vivo* studies in laboratory animals, type A proanthocyanidins (PAC), dose-dependently inhibited carrageenan-induced pleurisy, reduced lung damage, pleural exudate formation, infiltration of polymorphonuclear cells and blood levels of TNF-α, IL-1β, pro-inflammatory cytokines released by inflammation-activated macrophages. The anti-inflammatory effects of Black Currant proanthocyanidins are due to the inhibition of leukocyte infiltration explained, at least in part, by a significant decrease in endothelial adhesion molecules, (ICAM-1 and VCAM-1), compounds that are able to modulate the levels of TNF-α and IL-1β, induced by transcription of the Vascular Endothelial Growth Factor (VEGF). Currant also acts on immunocompetent cells, decreasing the production of IgE, without modifying the synthesis of other immunoglobulins. The synergy of the Black Currant phytocomplex activities can justify its use in local and systemic inflammatory syndromes, seasonal allergies, and bronchial asthma. Vitamin C is a water-soluble nutrient essential for the body that cannot be synthesized or stored by humans, and therefore must be taken daily. It is a powerful antioxidant with anti-inflammatory and immune support activities. Vitamin C, or ascorbic acid, is important for the synthesis of collagen, a protein necessary for the formation of the connective tissue of the skin, ligaments, and bones. It plays an important role in wounds and burns healing because it facilitates the formation of the scar connective tissue. It has antioxidant activity, protecting cells from free radicals oxidative stress. Vitamin C enhances neutrophil phagocytic capacity and oxidative killing, and supports lymphocyte proliferation and function, while antiviral activity against influenza virus is not due to its interaction with the H and N antigens of the virus, but it can be linked to its pro-oxidant effect. Therefore, the antiviral activity of ascorbic acid should occur regardless of the antigenic nature of the virus. The hypothesis that vitamin C may be useful in viral infections is based on two concepts, i.e. patients with acute infectious diseases have low levels of circulating vitamin C and that vitamin C has immunomodulatory properties in patients with viral infections, mainly increasing the production of α/β interferons and reducing that of pro-inflammatory cytokines. Several studies revealed that experimentally induced vitamin C deficiency reduces cellular and humoral immune responses. The same effect was demonstrated on different populations of immune cells both *in vivo* and in experimental models in humans. In clinical studies, vitamin C treatment of healthy subjects promoted and enhanced natural killer cell activity, lymphocyte proliferation and chemotaxis.

Currently there is a need to have preparations such as nutraceuticals or supplements whose intake contributes to resolving the typical symptoms of upper respiratory tract diseases, without having side effects, also in the case of long-term treatments.

Treatment with a product comprising Pelargonium sidoides, vitamin C, bromelin, quercetin and zinc has been proposed against flu and common colds. The product has shown results against throat, nose, and ear infections.

MOYO MACK ET AL: "Medicinal properties and conservation of Pelargonium sidoides", JOURNAL OF ETHNOPHARMACOLOGY, ELSEVIER IRELAND LTD, IE, vol. 152, no. 2, 21 January 2014 (2014-01-21), pages 243-255, discloses a composition comprising Pelargonium sidoides, vitamin C and bromelain for use in the treatment of respiratory tract infections.

A further need is to have preparations such as nutraceuticals or supplements whose intake also contributes to resolving typical symptoms of urinary tract infections with production of biofilms.

The general aim of the present invention is therefore the prevention and/or improvement of the clinical picture and/or of the symptoms connected to infections, in particular bacterial infections with biofilm production.

### SUMMARY OF THE INVENTION

In accordance with an aspect of the present invention, the inventors have found that some principles and enzymes interact synergistically in the prevention and in providing beneficial effects towards symptoms typical of upper respiratory and urinary tract diseases.

In a first aspect, therefore, the invention concerns a composition comprising bromelin, currant, pelargonium, vitamin C and resveratrol.

According to the invention, said principles can also be provided in combination with N-acetyl-I-cysteine.

The invention also concerns the composition comprising bromelin, currant, pelargonium, vitamin C and resveratrol, and optionally N-acetylcysteine, for use as a medicament.

According to a further aspect, the invention relates to the composition comprising bromelin, currant, pelargonium, vitamin C and resveratrol for use in the treatment of upper respiratory tract infections, urinary tract infections, joint infections, such as infections of prosthetic joints, in arthroplasty surgery, against urinary tract infections, such as cystitis and urocystitis, prostatitis and infections after radical prostatectomy, in particular infections of the upper respiratory tract and urinary tract.

In an advantageous aspect, the composition of the invention is suitable for use in pediatrics.

The invention therefore also concerns a supplement comprising the composition of the invention and excipients for use in the treatment, in particular, of the upper airways and urinary tracts, for both adults and pediatrics.

Advantageous aspects of the composition and supplement of the invention, such as amounts, posology and dosages will be indicated in detail in the detailed description, and the surprising synergistic effects resulting therefrom will be apparent in the subsequent experimental part.

In an advantageous aspect of the invention, the composition of the invention can be combined with at least one antibiotic for the treatment of bacterial infections of the upper respiratory tract and urinary tract.

The composition of the invention can, in fact, be administered alone or in combination, i.e. as a co-therapeutic agent in a fixed-dose combination or in a combination therapy of separately formulated active ingredients, with at least one antibiotic, preferably selected from the group consisting of amoxicillin, azithromycin, clarithromycin, ciprofloxacin, rifampicin, teicoplanin, tigecycline, linezolid, vancomycin, preferably amoxicillin.

The composition, or the supplement comprising the same, of the invention alone or advantageously in combination with at least one antibiotic resulted to be effective in the treatment of bacterial infections.

In fact, the composition of the invention, or the supplement comprising the same, allows to obtain at least one of the following advantages:
- bacterial biofilm disintegrating action,
- antimicrobial action,
- enhancement of antibiotic therapy,
- anti-inflammatory action.

In particular, the composition of the invention is therefore useful as a dietary and nutritional supplement, as a medicament for the prevention and treatment of infections, preferably bacterial, also with biofilm production. Said infections, preferably bacterial, are preferably pharyngotonsillitis, otitis, effusive otitis media, infectious and irritative inflammation of the upper airways, bacterial infections with biofilm production.

The composition of the invention resulted to be effective against diseases that are characterized by the formation of a bacterial biofilm. In fact, it resulted to be effective also in preventing formation of the same.

The composition object of the present invention therefore stands as a valid therapeutic tool for the management of infections, preferably bacterial, wherein the formation of biofilm hinders the action of any antibacterial agent.

Among the infections to be considered for a use of the composition, alone or in conjunction with at least one therapeutic agent, it is possible to preferably mention infections of prosthetic joints, in arthroplasty surgery, against urinary tract infections, such as cystitis and urocystitis, prostatitis and infections after radical prostatectomy. Co-administration with the composition in question therefore represents an effective therapy in preventing the risk of infections on artificial media.

### DESCRIPTION OF THE FIGURES

A brief description of the figures is provided below:
- Figure 1 shows the inhibition halos obtained against *Staphylococcus aureus,* after treatment with the composition (1), NAC diluted with lactose (2) and NAC alone (3) (experiment representative of three independent ones);
- Figure 2 shows the inhibition halos obtained against *Staphylococcus aureus* after treatment with the composition (1), NAC diluted with lactose (2), NAC alone (3) and a mixture not containing NAC (4) (experiment representative of three independent ones);
- Figures 3a and 3b show the degradation test of *Staphylococcus aureus* bacterial biofilm and corresponding graph, testing the composition and using NAC with lactose and NAC alone as control;
- Figure 4 shows the inhibition halos obtained against *Pseudomonas aeruginosa* after treatment with the composition (1), NAC diluted with lactose (2) and NAC alone (3) (experiment representative of three independent ones);
- Figure 5 shows the permeation profiles of NAC alone as compared with the composition;
- Figure 6 shows the antimicrobial activity against *Staphylococcus aureus* obtained by time-killing tests at 7, 17, 21 days of incubation. The formulation containing amoxicillin was used at a concentration corresponding to 0.01 mg/mL, while the composition was used at 0.3 mg/mL. A PBS saline solution was used as control;
- Figure 7 shows the synergistic action of the composition on bacterial growth inhibition in comparison with individual extracts thereof, and in comparison with a combination of Pelargonium, Bromelin and Vitamin C

### DETAILED DESCRIPTION OF THE INVENTION

The invention concerns a composition comprising bromelin, currant, pelargonium, vitamin C and resveratrol.

The composition of the invention is useful as a dietary and nutritional supplement, as a medicament for the prevention and treatment of bacterial infections, also with biofilm production.

In an advantageous aspect, the composition object of the present invention can also contain N-acetyl-I-cysteine or other cysteine derivatives.

As will be apparent from the experimental part, the inventors have noticed and ascertained a synergistic effect of the ingredients of the composition.

The composition therefore comprises bromelin, currant, pelargonium, vitamin C and resveratrol, preferably in an amount by weight per unit of formulation which is effective in the treatment of upper respiratory tract and urinary tract infections. The composition of the invention comprises the components in unit doses that fall within the following amount ranges:
a) bromelin: from 100 to 300 mg,
b) currant: from 50 to 150 mg,
c) pelargonium: from 30 to 120 mg,
d) vitamin C: from 50 to 200 mg
e) resveratrol: from 25 to 100 mg.

According to the invention, the composition also includes N-acetyl-I-cysteine, which can preferably be present in amounts from 150 to 400 mg as a unit dose.

According to a preferred form, the composition of the invention comprises the components in the following amounts per formulation unit:
a) N-acetyl-I-cysteine: 300 mg
b) bromelin: 200 mg
c) currant: 100 mg
d) pelargonium: 45 mg
e) vitamin C: 100 mg
f) resveratrol: 50 mg.

According to a further preferred form, the composition in tablets comprises the components in the following amounts per formulation unit:
a) N-acetyl-I-cysteine: 150 mg
b) bromelin: 150 mg
c) currant: 50 mg
d) pelargonium: 30 mg
e) vitamin C: 50 mg
f) resveratrol: 25 mg.

According to a still further preferred embodiment, the composition of the invention comprises the components in the following amounts per formulation unit:
a) N-acetyl-I-cysteine: 400 mg
b) bromelin: 300 mg
c) currant: 150 mg
d) pelargonium: 90 mg
e) vitamin C: 150 mg
f) resveratrol: 100 mg.

The invention therefore concerns the composition detailed above for use as a medicament.

According to a further aspect, the invention relates to the composition comprising bromelin, currant, pelargonium, vitamin C and resveratrol for use in the treatment of upper respiratory tract infections, urinary tract infections, joint infections, such as infections of prosthetic joints, in arthroplasty surgery, against urinary tract infections, such as cystitis and urocystitis, prostatitis and infections after radical prostatectomy, in particular infections of the upper respiratory tract and urinary tract.

In an advantageous aspect, the composition of the invention is suitable for use in pediatrics.

The invention therefore also concerns a supplement comprising the composition of the invention and excipients for use in the treatment of the upper respiratory tract and urinary tract, both for adults and in the pediatric field.

The composition and the supplement for use in the treatment of the upper airways and urinary tracts can preferably be administered by oral administration.

The compositions can therefore be in liquid, solid or semi-solid form and can be in the form of tablets, pearls, drops, sticks, syrups, sachets.

The compositions of the invention can be administered orally in the form of tablets, capsules, granulates, effervescent granulates, syrups or the like.

They can also be dissolvable in water to form inhalable solutions.

The compositions in solid form can comprise one or more additives such as starches, sugars, cellulose, diluents, granulating agents, lubricants, binders, disintegrating agents, stabilizers, emulsifiers, texturizers, plasticizers, wetting agents, thickeners, coatings. All these excipients allow to obtain the supplement of the invention in the desired pharmaceutical formulation.

The capsules can be coated with a gastro-resistant film, or they can be soft gel capsules.

Flavouring agents, preservatives, dyes or the like may also be present.

The active ingredients contained in the compositions object of the present invention can be combined or mixed as active ingredients and/or known excipients according to techniques of the pharmaceutical, and food or nutritional industries and prepared by methods known in the pharmaceutical or food industry.

The compositions object of the invention are also used as adjuvants in the prevention and treatment of infections/inflammations of the upper respiratory tract and urinary tract.

In an advantageous aspect of the invention, the composition of the invention may be combined with at least one antibiotic for the treatment of bacterial infections of the upper respiratory tract and urinary tract.

The composition of the invention can in fact be administered alone or in combination, i.e. as a co-therapeutic agent in a fixed-dose combination or in a combination therapy of separately formulated active ingredients, with at least one antibiotic, preferably selected from the group consisting of amoxicillin, azithromycin, clarithromycin, ciprofloxacin, rifampicin, teicoplanin, tigecycline, linezolid, vancomycin, preferably amoxicillin.

The composition, or the medicament or the supplement comprising the same, of the invention, alone or advantageously in combination with at least one antibiotic, resulted to be effective in the treatment of bacterial infections.

In fact, the composition of the invention, or the medicament or the supplement comprising the same, allows to obtain at least one of the following advantages:
- bacterial biofilm disintegrating action,
- antimicrobial action,
- antibiotic therapy enhancement,
- anti-inflammatory action.

In particular, the composition of the invention is therefore useful as a dietary and nutritional supplement, as a medicament for the prevention and treatment of infections, preferably bacterial, also with biofilm production. These infections, preferably bacterial, are preferably pharyngotonsillitis, otitis, effusive otitis media, infectious and irritative inflammation of the upper airways, infections affecting the joints, infections of synthetic or prosthetic media, infections of the urinary tract, joint infections and in general all infections caused by biofilm-producing bacteria.

The compositions object of the invention are therefore pharmaceutical preparations, nutraceuticals preparations or food supplements that can be introduced into the dietary regimen of an individual suffering from inflammatory symptoms, for simultaneous, separate, or subsequent use in the prophylaxis or treatment of a symptom related to upper respiratory tract and urinary tract inflammation and/or infection, also with biofilm production.

The composition object of the present invention is therefore a valid therapeutic tool for the management of infections, preferably bacterial, in which the formation of biofilm hinders the action of any antibacterial agent.

In this sense, the composition of the invention has also a preventive action since it avoids the formation of bacterial biofilm, and therefore avoids bacterial proliferation. Biofilm elimination and prevention of its formation are representative uses of the present composition against numerous diseases.

The inventors have therefore considered infections caused by bacteria grown in biofilms, due to their phenotypic variation and tolerance to antimicrobials, as they represent a worrying clinical issue to be solved with absolute priority. The bacteria inside the biofilm show greater resistance to disinfectants, antibiotics, phagocytes, and antibodies of the immune system.

The composition of the invention resulted to be effective against these bacteria, preventing the formation of biofilm.

Joint prosthesis infection represents a serious surgical complication. The infection rate for knee prostheses is 1-2%, while for hip prostheses a little less (0.3-1.7%). With the aging of the population, the number of joint replacement surgeries is bound to increase and with it the number of prosthetic infections. The incidence of infections associated with osteosynthesis devices (intramedullary nails, plates, screws, wires, transcutaneous fixation pins) varies from 1-2% for closed fractures to over 30% for open ones. In arthroplasty surgery, peri-operative antibiotic prophylaxis is carried out by parenteral route (on day of surgery followed by treatment for the following 3-5 days). Co-administration with the composition of the invention resulted to further prevent the risk of prosthetic infections and, at the same time, provide an effective treatment therapy where an infection with biofilm production occurs.

Bacterial proliferation in the bladder is the main cause of diseases affecting the urinary tract such as cystitis and urocystitis. Here, the majority of the bacteria responsible for cystitis-related infections are major biofilm producers, *E*. *Coli* being the first among all. Antibiotic treatment may not be a sufficient therapy due to the fact that colonies are well protected and difficult to be reached and exterminated by the drug. The synergistic treatment with the composition object of the present invention resulted to be a valid alternative to antibiotic therapy alone. The biofilm disintegration action of the composition, in fact, helps to expose the colony to the antimicrobial action of the antibiotic.

Like any major surgery, also radical prostatectomy involves risks and possible complications related to bacterial infections. The patient will be discharged with a bladder catheter which will be removed after at least one week. This phase is always accompanied by antibiotic therapy, which however can be ineffective due to the fact that the foreign bodies inserted in the ureter develop bacterial colonies protected by biofilm. Co-administration with the composition in question resulted to be an effective therapy in preventing the risk of infections on artificial media.

Prostatitis is a prostate disease that can have various causes, from mechanical to bacterial ones. Prostate infection is a common pathological condition in many men, in both young and old age, and causes annoying symptoms that compromise the patient's sexual life and urination. The main therapies for management of prostatic health are aimed at controlling the gland volume, which causes very acute pain when increased. The same importance should be paid to the management of prostatic bacterial growth when the prostatitis is of a bacterial type. In this case, the therapy always involves the use of antibiotics, which, however, are often ineffective, as the colony is protected by bacterial biofilms. As for the previous applications, the co-administration therapy with antibiotic and composition resulted to be the most complete strategy for management of prostate infection.

The synergistic biological action exerted by the composition of the invention is surprising because it is attributable to a multitarget action exerted by the biologically active components on the cellular inflammatory and redox pathways against many diseases. A synergistic action means an activity that is greater than the sum of the activities of the single active ingredient (additive action), i.e. the active ingredients of the composition interact with each other enhancing the effects, as will be apparent from the following experimental part.

### EXPERIMENTAL PART

The following composition was tested:
- N-acetyl cysteine (NAC)
- Bromelin
- Currant
- Pelargonium
- Ascorbic acid
- Resveratrol

A Gram-positive bacterium, *Staphylococcus aureus* (ATCC 6538) and a Gram-negative bacterium, *Pseudomonas aeruginosa* (ATCC 27853) were used to determine the antimicrobial capacity of the composition under examination.

### DISC DIFFUSION TEST

Each bacterium was inoculated, individually, in 10 mL of Mueller Hinton Broth (MHB) and incubated overnight at 37°C under shaking.

Following spectrophotometric reading from the overnight cultures at 600 nm, two cultures of S. *aureus* and *P. aeruginosa* were prepared, each with 0.8 × 10⁸ cells/mL.

A cotton swab was dipped into each bacterial suspension so as to inoculate the culture in a 90 mm plate of Mueller Hinton Agar by manually swiping over the entire surface of the medium, taking care that there are no areas without inoculum. Within 30 minutes from seeding, the powders were positioned at a distance such as to avoid any halos overlapping.

5, 10 and 15 mg of the composition were tested:
- N-acetyl-cysteine: 300 mg,
- Bromelin: 200 mg,
- Ribes nigrum: 100 mg,
- Pelargonium sidoides: 45 mg,
- Vitamin C: 100 mg,
- Resveratrol: 50 mg,
using N-acetyl cysteine diluted with lactose and pure N-acetyl cysteine as controls. The plates, thus prepared, were incubated overnight in aerobiosis at 35 ± 1°C. After incubation, the inhibition halos were measured, and the images were processed. The experiments were carried out in triplicate.

### BIOFILM ASSAY

S. *aureus* was inoculated in 10 mL of Mueller Hinton Broth (MHB) and incubated overnight at 37°C under shaking. A sterile 96-well flat-bottom polystyrene plate was filled with 200 µL of culture containing 2× 10⁶ cell/mL.

Different amounts of formulation were tested, suitably dissolved in DMSO (chosen because the formulation was not soluble in either water or ethanol).

Amounts of formulation containing increasing amounts of NAC were tested: 1.8; 9; 36; 72; 144 µg of NAC, in 200 µL of bacterial culture. Powders of pure N-acetyl cysteine and N-acetyl cysteine diluted with lactose were used as controls.

The plate was incubated at 37°C for 48 hours. After incubation, non-adherent cells were removed, and the wells were washed with 200 µL of phosphate buffered saline (pH 7.2) to remove floating bacteria.

The biofilms formed by bacteria adhering to the wells were stained with a 3% solution of crystal violet (CV).

The plate was placed at 37°C, for 1 hour, without shaking.

Subsequently, the crystal violet was removed, and the wells were washed three times in order to acquire a photographic image. After adding 200 µL of a 33% acetic acid solution to each well, the optical density of the stained adherent biofilm was measured using a plate spectrophotometer at a wavelength of 600 nm.

The experiments were carried out in triplicate.

### RESULTS

### STAPHYLOCOCCUS AUREUS 6538

### DISC DIFFUSION

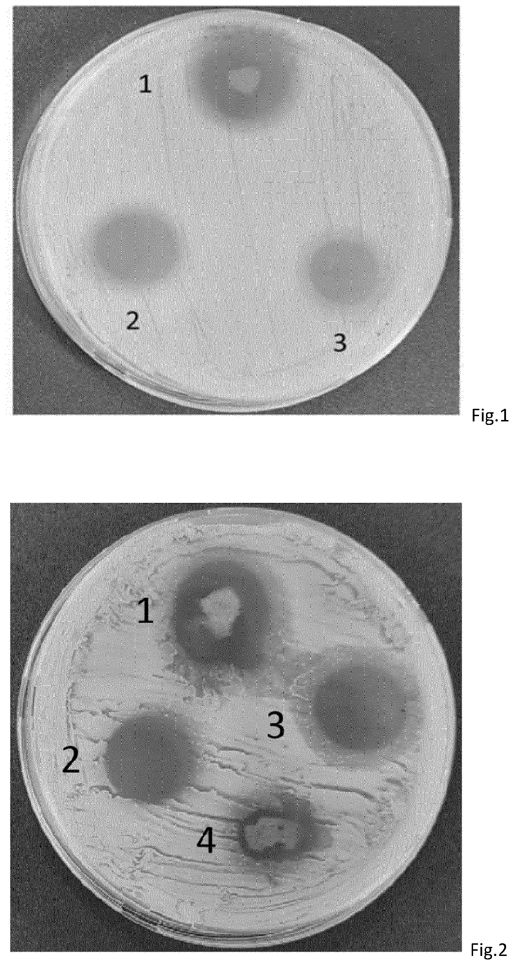

Figure 1 shows the inhibition halos obtained against *Staphylococcus aureus,* testing 15 mg of the formulation, and using NAC diluted with lactose and NAC alone as controls.

**Table 1 below shows the amount of powder placed on the plate, the amount of NAC in the sample, diameter, area, diameter and area normalized with respect to the amount of NAC, and diameter and area percentage increase with respec to NAC alone.**

| | **Powder (mg)** | **NAC (mg)** | **Diamete r (mm)** | **Area (mm²)** | **Normalized NAC amount** | **Diameter (mm)** | **Area (mm²)** | **Δ Diam eter** | **Δ Area** |
|---|---|---|---|---|---|---|---|---|---|
| Formulation | 14.97 | 5.65 | 18.7 | 287.8 | 5.78 | 19.1 | 294.4 | 35 | 73 |
| NAC + lactose | 14.94 | 5.64 | 16.3 | 198.7 | 5.78 | 16.7 | 203.6 | 7 | 9 |
| NAC | 5.78 | 5.78 | 14.2 | 170.1 | 5.78 | 14.2 | 170.1 | | |

As shown in Table 1, there is an increase in the inhibition halo measurable both by diameter and area.

Precisely, the formulation led to an increase in diameter equal to 35% and an increase in area equal to 73%, when compared to NAC alone as control. The test also shows a slight increase in the inhibition halo for the NAC + Lactose composition. This is not a sign of an increase in the antibacterial action of NAC + Lactose but is due to a better dissolution and spreading of the NAC powder on the plate thanks to the lactose.

Figure 2 shows the inhibition halos of an experiment carried out by testing also the powder containing all the components of the formulation, except for NAC, to evaluate a synergistic effect of the composition (4). As can be seen, the composition of the components in the absence of NAC provides a statistically detectable inhibition halo, indication of the antibacterial activity of the composition of components, which is expressed even better when NAC is added to the composition.

The light grey portion in the centre of the inhibition zones is represented by the herbal extracts not completely solubilized in the culture medium. It should be considered that the presence of herbal extracts insoluble in organic soil does not compromise the antibacterial action of the composition.

### BIOFILM ASSAY

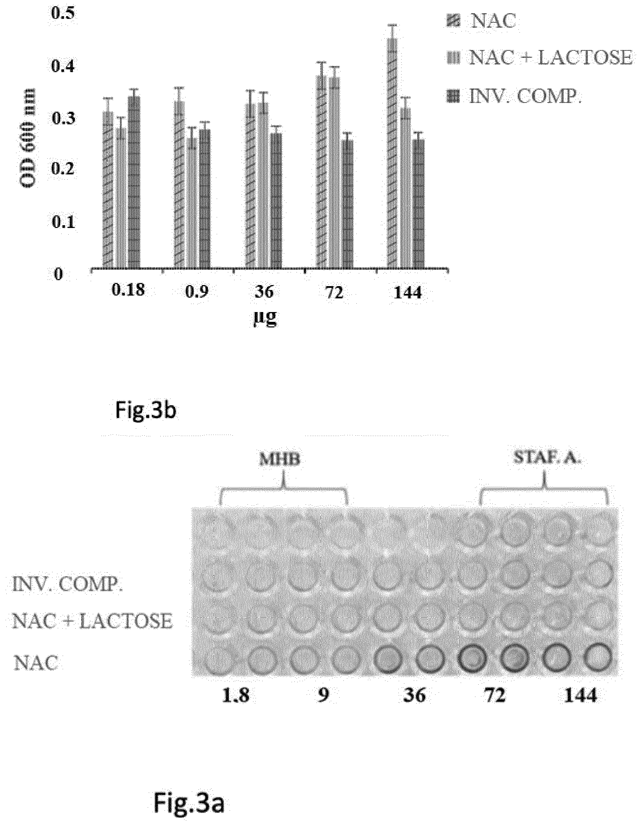

The evaluation of the bacterial biofilm reduction was performed on *Staphylococcus aureus,* given the best results obtained from the diffusion test on plate. The formulation containing increasing concentrations of NAC was tested. As can be seen from the bleaching of the wells (Figures 3a and 3b), it is possible to highlight a significant reduction in the production of bacterial biofilm in the wells treated with the formulation.

### PSEUDOMONAS AERUGINOSA 27853

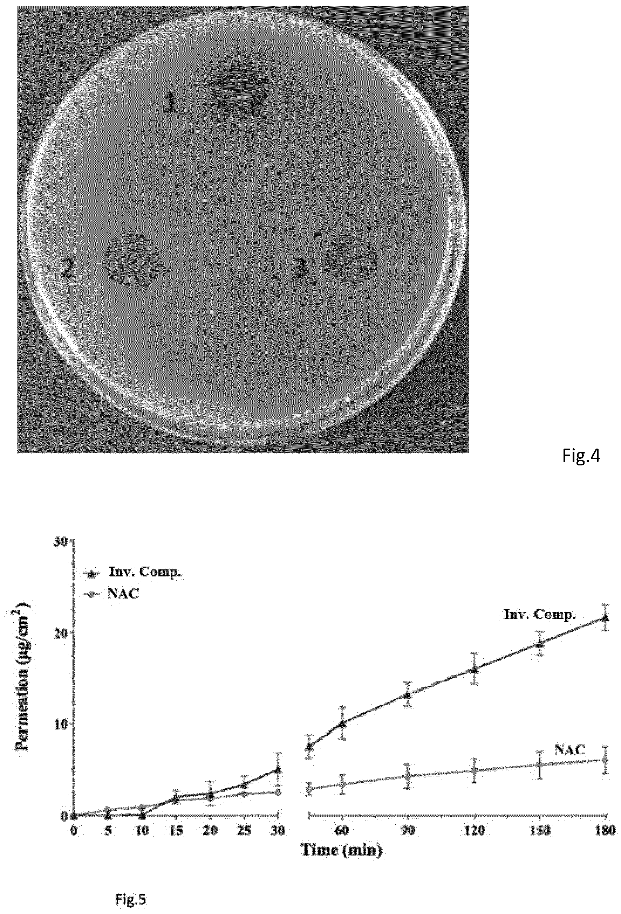

Disc diffusion tests performed on the Gram-negative bacterium *P. aeruginosa* (Figure 4) showed an effect of the formulation also against the Gram-negative bacterium.

The formulation, in fact, increased the diameter of the inhibition halo by 15% and an increase in the inhibition area equal to 38%, as compared to NAC alone as control. Also in this case it was possible to observe an effect of lactose in the NAC+Lactose composition which leads to an increase in the inhibition halo (Table 2).

**Table 2 shows the amount of powder placed on the plate, the amount of NAC in the sample, diameter, area, diameter and area normalized with respect to the amount of NAC, and diameter and area percentage increase compared to NAC alone:**

| | **Powder (mg)** | **NAC (mg)** | **Diameter (mm)** | **Area (mm²)** | **Normalized NAC amount** | **Diameter (mm)** | **Area (mm²)** | **Δ Diameter** | **Δ Area** |
|---|---|---|---|---|---|---|---|---|---|
| Formulation | 14.86 | 5.6 | 11.1 | 99.7 | 5.5 | 11.17 | 98.63 | 8 | 29 |
| NAC + lactose | 15.17 | 5.7 | 10.8 | 101.2 | 5.5 | 10.47 | 98.1 | 2.74 | 28 |
| NAC | 5.55 | 5.5 | 10.2 | 76.7 | 5.5 | 10.19 | 76.7 | | |

### PREPARATION OF THE ARTIFICIAL MUCUS

The artificial mucus was prepared by dispersing 15 g of hydroxyethylcellulose in distilled water under stirring and heating, until a homogeneous solution is obtained. After cooling, 4 g of deoxyribonucleic acid (DNA), 5 g of mucin, 5.9 mg of diethylenetriaminepentaacetic acid (DTPA), 20.0 mL of an RPMI 1640 amino acids solution, 5 mL of egg yolk emulsion, 5.0 g NaCl and 2.2 g of KCl were added to the solution, to obtain a final volume of 1 L.

The pH of the resulting solution was brought to 7.0 ± 0.1 by means of a NaOH solution.

The mucus thus obtained was allowed to equilibrate at 25°C for 12 h before analysis.

### PERMEATION STUDIES THROUGH THE ARTIFICIAL MUCUS

The permeation studies were carried out using Franz-type vertical diffusion cells (Hanson research corporation, CA, USA) having an exposed area of 0.60 cm.

The cell serving as the receptor compartment was filled with 5 mL of distilled water (pH 6.5).

The receiving solution was thermostated at 37°C and maintained under magnetic stirring at 150 rpm by means of a Teflon-coated magnetic stir bar kept in the receptor compartment.

A nitrocellulose membrane (25 mm diameter, Merk KGaA, Darmstadt, Germany) was placed as a separating septum between the donor and the receptor compartments and coated, on the donor side, by a thin layer of artificial mucus of about 3mm.

The powders to be tested were subsequently placed on the mucus layer and 200 µL aliquots of the solution present in the receptor compartment were sampled at regular time intervals, and subsequently analysed by HPLC for determination of the permeate.

After each sampling, the same amount of solution withdrawn was replaced with the same volume of distilled water. The permeation per unit area (Q) was calculated for each time interval.

Permeation data were reported as the amount of permeated substance per unit area at each single timepoint.

All permeation experiments were tested in triplicate, expressing the results as mean ± standard deviation.

As shown in figure 5, where NAC permeation profiles have been reported in comparison with the composition, it is possible to observe how the permeation of the composition is clearly better than that of NAC alone.

In fact, the permeation of NAC alone is very low (about 1.8 µg/cm² after 30 min and 4.4 µg/cm² after 180 min), whereas when present in the composition of extracts, the permeation values increase considerably (about 2.5 µg/cm² after 30 min and 21.6 µg/cm² after 180 min).

Antimicrobial activity tests against *Staphylococcus aureus* and *Pseudomonas aeruginosa* have shown that the formulation is able to inhibit the growth of both bacteria with a greater effect on the Gram-positive, S. *aureus.*

Furthermore, the formulation under study also demonstrated a statistically significant effect on the degradation of S. *aureus* bacterial biofilm. In experiments of permeation through artificial mucus, the composition of NAC and natural extracts (product object of the invention) proved to be considerably more effective than NAC alone.

### ANTIMICROBIAL EFFECTIVENESS OF THE COMPOSITION OVER TIME.

Antibiotic efficacy studies over time were carried out by means of a "time-killing assay" carried out against the *Staphylococcus aureus* strain A170 in co-culture with HeLa cells (immortalized human cells). The inoculation of S. *aureus* was carried out at a standard concentration of 0.5 McFarland (approximately 1.5 × 10⁸ colony forming units (CFU)/mL) in MHB.

The bacterial colonies were inoculated in wells (200 µL/well) containing the cell cultures and, after a co-culture overnight at 37°C, they were treated with amoxicillin and the different samples to be tested, and with a saline solution, used as a control. After incubation in co-culture the viable bacterial colonies were calculated at different time intervals (7, 14, 21 days), by separating the bacterial colonies from the cells, and making serial dilutions on MHA and subsequently incubating at 35°C. The number of CFUs recovered was then reported against time. The experiments were carried out in triplicate for each single experimental point reported.

### Time killing assay

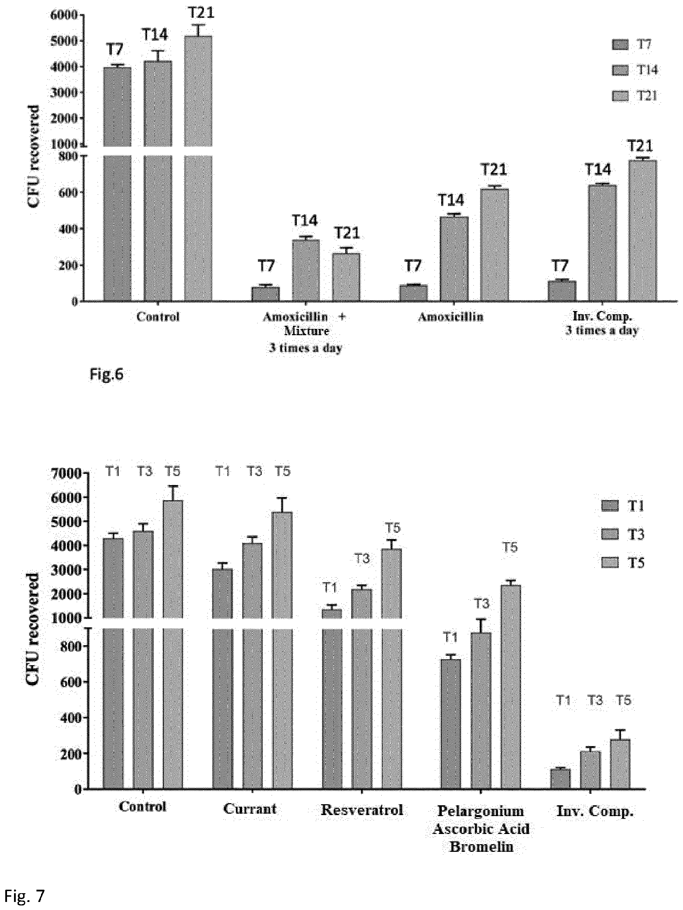

Figure 6 shows the colony forming units (CFU recovered = number of colony forming units of S. *aureus.* Mean ± SD; (n = 6)) related to *Staphylococcus aureus,* after exposures of bacteria in co-culture with HeLa cells, previously contaminated with appropriate concentrations of microorganisms, treated only with amoxicillin and lactose (control), amoxicillin and composition.

As reported in Figure 6, treatment with amoxicillin in combination with the composition showed a statistically significant increase in the bacterial growth inhibitory power in the conditions tested as compared to amoxicillin alone. Furthermore, the figure shows how, after the seventh day of monitoring, also treatment with the composition alone for three times a day, has a significant action against S. *aureus.*

### CONCLUSIONS

Tests of antimicrobial activity against *Staphylococcus aureus* and composition administered 3 times a day in addition to the active ingredient amoxicillin showed a significant increase in the efficacy of joint administration both with respect to the composition alone and to amoxicillin alone for each time interval considered.

### EVALUATION OF THE SYNERGISM OF THE COMPOSITION OF THE INVENTION.

The Gram-positive bacterium, *Staphylococcus aureus* (ATCC 6538) was used to determine the synergistic antimicrobial capacity of the composition of the invention with respect to the individual extracts and products of the prior art.

Bacteria were inoculated in 10 mL of Mueller Hinton Broth (MHB) and incubated overnight at 37°C under shaking.

Following spectrophotometric reading from the overnight cultures at 600 nm, two cultures were prepared, each with 0.8 × 10⁸ cells/mL of S. aureus.

After incubation in co-culture the viable bacterial colonies were calculated at different time intervals by separating the bacterial colonies from the cells and performing serial dilutions on MHA and subsequently incubating at 35°C.

The number of CFUs recovered was then reported against time. The experiments were carried out in triplicate for each single experimental point reported.

Figure 7 shows the colony forming units (CFU recovered = number of colony forming units of S. *aureus.* Mean ± SD; (n = 6)) related to *Staphylococcus aureus,* after exposures of the bacteria in co-culture with:
1) -Pelargonium sidoides
   - Vitamin C
   - Bromelin
2) - Ribes nigrum
3) - Resveratrol
4) - Pelargonium sidoides
   - Vitamin C
   - Bromelin
   - N-Acetyl-cysteine
   - Ribes nigrum
   - Resveratrol

As can be seen in Fig. 7, the treatment with the composition in question showed a statistically significant increase in the bacterial growth inhibition power in the conditions tested with respect to Ribes alone, Resveratrol alone, the mixture consisting of Pelargonium, Ascorbic Acid and Bromelin, in the same amounts. Furthermore, Figure 7 shows how in the first day of monitoring, the treatment with the composition of the invention has a remarkable action as % of inhibition against S. *aureus* which reaches 98% compared to 30% of Ribes, 56% of Resveratrol and 84% of the mixture consisting of Pelargonium, Vitamin C and Bromelin. This showed that the synergistic action of the composition in reducing bacterial proliferation is greater than the additive sum of the individual contributions of extracts thereof or mixtures of the same.

## Claims

1. A composition comprising bromelin, currant, pelargonium, vitamin C and resveratrol.

2. The composition according to claim 1, wherein said composition further comprises N-acetyl-I-cysteine.

3. The composition according to claim 1, wherein said composition comprises:
a) bromelin: from 100 to 300 mg,
b) currant: from 50 to 150 mg,
c) pelargonium: from 30 to 120 mg,
d) vitamin C: from 50 to 200 mg
e) resveratrol: from 25 to 100 mg.

4. The composition according to any one of claims 2 to 3, wherein N-acetyl-I-cysteine is in an amount from 150 to 400 mg.

5. The composition according to claim 3 o 4, wherein said composition comprises:
a) N-acetyl-I-cysteine: 300 mg
b) bromelin: 200 mg
c) currant: 100 mg
d) pelargonium: 45 mg
e) vitamin C: 100 mg
f) resveratrol: 50 mg.

6. The composition according to claim 3 o 4, wherein said composition comprises:
a) N-acetyl-I-cysteine: 150 mg
b) bromelin: 150 mg
c) currant: 50 mg
d) pelargonium: 30 mg
e) vitamin C: 50 mg
f) resveratrol: 25 mg.

7. The composition according to claim 3 o 4, wherein said composition comprises:
a) N-acetyl-I-cysteine: 400 mg
b) bromelin: 300 mg
c) currant: 150 mg
d) pelargonium: 90 mg
e) vitamin C: 150 mg
f) resveratrol: 100 mg.

8. A composition according to any one of claims 1 to 7 for use as a medicament.

9. A supplement comprising the composition according to any one of claims 1 to 7 and excipients suitable for the final formulation.

10. A composition according to any one of claims 1 to 7 or a supplement according to claim 9 for use in the treatment of upper respiratory tract infections, urinary tract infections, joint infections such as infections of prosthetic joints, in arthroplasty surgery, against urinary tract infections, such as cystitis and urocystitis, prostatitis and infections after radical prostatectomy.

11. The composition or supplement for use according to claim 10, wherein the composition or supplement is in a liquid, solid or semi-solid form suitable for oral administration.

12. The composition or supplement for use according to claim 11, wherein the composition or supplement is in the form of tablets, capsules, granulates, effervescent granulates, pearls, drops, sticks, syrups, and sachets.

13. The composition or supplement for use according to any one of claims 10 to 12 as a co-therapeutic agent in combination with at least one antibiotic, preferably selected from the group consisting of amoxicillin, azithromycin, clarithromycin, ciprofloxacin, rifampicin, teicoplanin, tigecycline, linezolid, vancomycin, preferably amoxicillin.

14. The composition or supplement for use according to any one of claims 10 to 13, wherein the treatment takes place against pharyngotonsillitis, otitis, effusive otitis media, infectious and irritative inflammation of the upper airways, bacterial infections of the airways and/or urinary tracts, also with biofilm production.

## Patentansprüche

1. Eine Zusammensetzung, die Bromelin, Johannisbeere, Pelargonium, Vitamin C und Resveratrol enthält.

2. Zusammensetzung nach Anspruch 1, wobei die Zusammensetzung außerdem N-Acetyl-I-Cystein enthält.

3. Zusammensetzung zur Verwendung nach Anspruch 1, wobei die Zusammensetzung Folgendes umfasst:
a) Bromelin: von 100 bis 300 mg,
b) Johannisbeere: von 50 bis 150 mg,
c) Pelargonium: von 30 bis 120 mg,
d) Vitamin C: von 50 bis 200 mg
e) Resveratrol: von 25 bis 100 mg.

4. Zusammensetzung nach einem der Ansprüche 2 bis 3, wobei N-Acetyl-I-Cystein in einer Menge von 150 bis 400 mg vorhanden ist.

5. Zusammensetzung nach Anspruch 3 oder 4, wobei die Zusammensetzung umfasst:
a) N-Acetyl-I-Cystein: 300 mg
b) Bromelin: 200 mg
c) Johannisbeere: 100 mg
d) Pelargonium: 45 mg
e) Vitamin C: 100 mg
f) Resveratrol: 50 mg.

6. Zusammensetzung nach Anspruch 3 oder 4, wobei die Zusammensetzung umfasst:
a) N-Acetyl-I-Cystein: 150 mg
b) Bromelin: 150 mg
c) Johannisbeere: 50 mg
d) Pelargonium: 30 mg
e) Vitamin C: 50 mg
f) Resveratrol: 25 mg.

7. Zusammensetzung nach Anspruch 3 oder 4, wobei die Zusammensetzung umfasst:
a) N-Acetyl-I-Cystein: 400 mg
b) Bromelin: 300 mg
c) Johannisbeere: 150 mg
d) Pelargonium: 90 mg
e) Vitamin C: 150 mg
f) Resveratrol: 100 mg.

8. Zusammensetzung nach einem der Ansprüche 1 bis 7 zur Verwendung als Medikament.

9. Zusatz umfassend die Zusammensetzung nach einem der Ansprüche 1 bis 7 und Trägerstoffe, die für die endgültige Formulierung geeignet sind.

10. Zusammensetzung nach einem der Ansprüche 1 bis 7 oder ein Zusatz nach Anspruch 9 zur Verwendung bei der Behandlung von Infektionen der oberen Atemwege, Harnwegsinfektionen, Gelenkinfektionen, wie Infektionen von Prothesengelenken, bei Gelenkersatzoperationen, gegen Harnwegsinfektionen, wie Zystitis und Urozystitis, Prostatitis und Infektionen nach radikaler Prostatektomie.

11. Zusammensetzung oder Zusatz zur Verwendung gemäß Anspruch 10, wobei die Zusammensetzung oder Zusatz in einer flüssigen, festen oder halbfesten Form vorhanden ist, die für eine orale Verabreichung geeignet ist.

12. Zusammensetzung oder Zusatz zur Verwendung nach Anspruch 11, wobei die Zusammensetzung oder Zusatz in Form von Tabletten, Kapseln, Granulaten, Brausegranulaten, Perlen, Tropfen, Stick, Sirup und Beuteln vorhanden ist.

13. Zusammensetzung oder Zusatz zur Verwendung nach einem der Ansprüche 10 bis 12 als Co-Therapeutikum in Kombination mit mindestens einem Antibiotikum, vorzugsweise ausgewählt aus der Gruppe bestehend aus Amoxicillin, Azithromycin, Clarithromycin, Ciprofloxacin, Rifampicin, Teicoplanin, Tigecyclin, Linezolid, Vancomycin, vorzugsweise Amoxicillin.

14. Zusammensetzung oder Zusatz zur Verwendung nach einem der Ansprüche 10 bis 13, wobei die Behandlung gegen Pharyngotonsillitis, Otitis, effusive Otitis media, infektiöse und irritative Entzündungen der oberen Atemwege, bakterielle Infektionen der Atemwege und/oder Harnwege, auch mit Biofilmbildung, erfolgt.

## Revendications

1. Composition comprenant de la broméline, de la groseille, du pélargonium, de la vitamine C et du resvératrol.

2. Composition selon la revendication 1, dans laquelle ladite composition comprend en outre de la N-acétyl-I-cystéine.

3. Composition selon la revendication 1, dans laquelle ladite composition comprend :
a) de la broméline : de 100 à 300 mg,
b) de la groseille : de 50 à 150 mg,
c) du pélargonium : de 30 à 120 mg,
d) de la vitamine C : de 50 à 200 mg
e) du resvératrol : de 25 à 100 mg.

4. Composition selon l'une quelconque des revendications 2 à 3, dans laquelle la N-acétyl-I-cystéine représente une quantité comprise entre 150 et 400 mg.

5. Composition selon la revendication 3 ou 4, dans laquelle ladite composition comprend :
a) de la N-acétyl-I-cystéine : 300 mg
b) de la broméline : 200 mg
c) de la groseille : 100 mg
d) du pélargonium : 45 mg
e) de la vitamine C : 100 mg
f) du resvératrol : 50 mg

6. Composition selon la revendication 3 ou 4, dans laquelle ladite composition comprend :
a) de la N-acétyl-I-cystéine : 150 mg
b) de la broméline : 150 mg
c) de la groseille : 50 mg
d) du pélargonium : 30 mg
e) de la vitamine C : 50 mg
f) du resvératrol : 25 mg

7. Composition selon la revendication 3 ou 4, dans laquelle ladite composition comprend :
a) de la N-acétyl-I-cystéine : 400 mg
b) de la broméline : 300 mg
c) de la groseille : 150 mg
d) du pélargonium : 90 mg
e) de la vitamine C : 150 mg
f) du resvératrol : 100 mg

8. Composition selon l'une quelconque des revendications 1 à 7 à utiliser comme médicament.

9. Supplément comprenant la composition selon l'une quelconque des revendications 1 à 7 et des excipients appropriés pour la formulation finale.

10. Composition selon l'une des revendications 1 à 7 ou supplément selon la revendication 9 pour le traitement des infections des voies respiratoires supérieures, des infections des voies urinaires, des infections articulaires telles que les infections des prothèses articulaires, dans la chirurgie d'arthroplastie, contre les infections des voies urinaires, telles que la cystite et l'urocystite, la prostatite et les infections après prostatectomie radicale.

11. Composition ou supplément à utiliser selon la revendication 10, dans laquelle/lequel la composition ou le supplément se présente sous une forme liquide, solide ou semi-solide adaptée à l'administration orale.

12. Composition ou supplément à utiliser selon la revendication 11, dans laquelle/lequel la composition ou le supplément se présente sous forme de comprimés, de capsules, de granulés, de granulés effervescents, de perles, de gouttes, de bâtonnets, de sirops et de sachets.

13. Composition ou supplément à utiliser selon l'une des revendications 10 à 12 en tant qu'agent cothérapeutique en association avec au moins un antibiotique, de préférence choisi dans le groupe constitué de l'amoxicilline, l'azithromycine, la clarithromycine, la ciprofloxacine, la rifampicine, la teicoplanine, la tigécycline, le linézolide, la vancomycine, de préférence l'amoxicilline.

14. Composition ou supplément à utiliser selon l'une des revendications 10 à 13, dans laquelle/lequel le traitement s'effectue contre la pharyngotonsillite, l'otite, l'otite moyenne effusive, l'inflammation infectieuse et irritative des voies respiratoires supérieures, les infections bactériennes des voies respiratoires et/ou des voies urinaires, y compris avec production de biofilm.
